# EUROPEAN PATENT SPECIFICATION

(11) **EP 1 108 698 B1**
(45) Date of publication and mention of the grant of the patent: **05.07.2006**
(21) Application number: 00204593.8
(22) Date of filing: 18.12.2000
(51) Int. Cl.: C04B 38/06, A61L 27/12

(54) **Porous ceramic body**
Keramischer Schaumkörper
Corps céramique poreux

(30) Priority: 16.12.1999 EP 99204378
(43) Date of publication of application: 20.06.2001
(73) Proprietor: IsoTis B.V., 3723 MB Bilthoven (NL)
(72) Inventor: Li, Shihong, 3564 AE Utrecht (NL); de Groot, Klaas, 2101 EL Heemstede (NL); Layrolle, Pierre Jean François, 3513 CT Utrecht (NL); van Blitterswijk, Clemens Antoni, 3467 PD Hekendorp (NL); de Wijn, Joost Robert, 6522 BP Nijmegen (NL)
(74) Representative: Prins, Adrianus Willem

(56) References cited:
- GB-A- 1 033 560
- GB-A- 2 317 887
- US-A- 4 894 194
- CHEMICAL ABSTRACTS, vol. 102, no. 14, 8 April 1985 (1985-04-08) Columbus, Ohio, US; abstract no. 118385g, TOYO RUBBER INDUSTRY: page 310; XP002138030 & JP 59 190250 A (ID.) 29 October 1984 (1984-10-29)
- CHEMICAL ABSTRACTS, vol. 104, no. 18, 5 May 1986 (1986-05-05) Columbus, Ohio, US; abstract no. 154446c, T. NAGAI ET AL.: page 345; XP002138031 & JP 60 200874 A (ID) 11 October 1985 (1985-10-11)
- PATENT ABSTRACTS OF JAPAN vol. 1997, no. 08, 29 August 1997 (1997-08-29) & JP 09 108567 A (SHARP CORP.), 28 April 1997 (1997-04-28)
- PATENT ABSTRACTS OF JAPAN vol. 018, no. 093 (C-1166), 16 February 1994 (1994-02-16) & JP 05 294752 A (JAPAN STEL WORKS LTD)

## Description

The invention relates to a method of preparing a porous ceramic body and to a body obtainable by said method. The invention further relates to the use of said body as a scaffold in tissue engineering.

Regeneration of skeletal tissues has been recognized as a new means for reconstruction of skeletal defects arising from abnormal development, trauma, tumors and other conditions requiring surgical intervention. Autologous bone grafting is considered the gold standard of bone transplantation with superior biological outcomes. However, autologous bone stocks are limited and often insufficient, particularly when large skeletal defects are encountered. Allografts are used as alternative materials but are associated with immunologically mediated complications and risks of disease transmission. Additional disadvantages of autograft and allograft materials include their limited potential for molding or shaping to achieve an optimum fit with bone voids.

As surgical techniques and medical knowledge continue to advance, there is an increasing demand for synthetic bone replacement materials, resulting from the limited supply of autograft materials and the health risks associated with the use of allografts. Hydroxyapatite has been investigated for use in the osseous environment for over 20 years, and the biocompatibility of the ceramic and its osteoconductive behavior is well established. Since porous HA is more resorbable and more osteoconductive than dense HA, there is an increasing interest in the development of synthetic porous hydroxyapatite (HA) bone replacement materials for the filling of both load-bearing and non-load-bearing osseous defects. Such technology could have the potential for restoration of vascularization and complete penetration of osseous tissue throughout the repair site.

Variation of the scaffold design as three-dimensional superstructures has been demonstrated as an approach to optimize the functionality of bone regeneration materials so that these materials may be custom designed for specific orthopedic applications in the form of void fillers, implants, or implant coatings. In attempt to develop a skeletal cell and tissue carrier, which could provide optimal spatial conditions for cell migration and maintenance by the arrangement of structural elements such as pores and fibers, the feasibility of using "live" material is under investigation. Such live material could take the form of an open-porous implant system together with living tissue. In other words, this is so-called hard tissue engineering.

The most traditional way of preparing a porous HA ceramic is to use a foaming agent like hydrogen peroxide (H₂O₂). In detail, an HA slurry is made by mixing HA powder with water and a H₂O₂ solution. Then, samples of the slurry are put in oven, under elevated temperature. H₂O₂ decomposes and O₂ is released from the bulk material, leaving a porous structure. Until today, this technique is still widely used in both clinical applications and research areas. However, porous ceramics made by this H₂O₂ method has an intrinsic shortcoming: it possesses only "laminar porosity". In other words, the pores are interconnected mostly in a laminar way, so there is no truly three-dimensional interconnected structure.

Slip-casting is another way of synthesizing porous ceramics. The manufacturing route comprises preparing an HA slurry (slip) by mixing HA powder under stirring with water, a deflocculant and binder agents. In this slurry, a kind of foam (sponge) is immersed and pressed. As a result, the slurry will be sucked into the foam. A layer of ceramic will be coated on all the struts of the sponge after removing the extra slurry by squeezing the samples. Then the samples will be dried in microwave oven and finally sintered in a furnace. This method is often referred to as a positive replica method.

Slip-casted materials are highly porous; they have a reticulate structure. However, due to the inner flaws in the ceramic, which are left after the sponge is burnt off, the strength of the material can not be increased to meet the requirement of tissue engineering application.

Meanwhile, coral HA has gained a wide interest in biomaterial spheres. An example of such a material is Interpore®, which has a high porosity and excellent microporous surface structure. However, it is an expensive material and, more importantly, its mechanical strength is insufficient for tissue engineering applications.

In Chemical Abstracts, vol. 102, no. 14, 8 April 1985, abstract no. 118385g, a method for the manufacture of a porous ceramic body is disclosed, in which a polyalkylene polyol (A) and an organic isocyante (B) are mixed to give a premix which is mixed with thick ceramic slurries prepared by mixing a crosslinking agent (C) and a water-base ceramic slurry to make A + B + C of the ceramic raw material, poured into a mold, and foamed to give an urethane foamed body which is dried and fired to obtain a porous ceramic body.

In Chemical Abstracts, vol. 104, no. 18, 5 May 1986, abstract no. 154446c, a preparation of foamed ceramic bodies is described, wherein a ceramic raw material is mixed with water, a surfactant and/or tannic acid or its salt to form a ceramic slurry, then mixing with urethane prepolymer prepared by allowing poly(oxyalkylene)polyol containing an ethylene oxide unit, poring in a mold, allowing to foam, drying, and firing.

The British patent application 2 317 887 discloses porous ceramic articles which may be used in bone cell culture. The articles are prepared by forming a dispersion comprising a liquid carrier and the particles and a polymerisable monomeric material; forming a foam of the dispersion; polymerising the foamed structure; drying the structure to remove the liquid carrier and provide a solid article having pores derived from the bubbles; and firing the article to remove the organic binder and provide a ceramic bond.

US patent 4,894,194 discloses a method for molding ceramic powders comprising forming a slurry mixture including ceramic powder, a dispersant for the metal-containing powder, and a monomer solution. The slurry mixture is transferred to a mold, and the mold containing the slurry mixture is heated to polymerize and crosslink the monomer and form a firm polymer-solvent gel matrix. The solid product may be removed from the mold and heated to first remove the solvent and subsequently remove the polymer, whereafter the product may be sintered.

In summary, there are several known methods of preparing porous ceramics. However, there are specific requirements for porous ceramic which are to be used for skeletal regeneration, hard tissue repairing, and even for hard tissue engineering purpose. For bony tissue ingrowth, it is accepted that the pore size should be in the range of 100 to 300 microns, and the pores should be fully interconnected. This specification gives rise to a desire for a more suitable porous ceramic (tissue carrier or 3-D scaffold).

The present invention provides an improved method for preparing a porous ceramic body. The method leads to a ceramic body having interconnected pores of a controllable pore size. Furthermore, the mechanical properties of the ceramic body are superior to those of ceramic bodies prepared by the above discussed, known methods. Particularly its compressive strength is much higher.

A process for preparing a porous ceramic body according to the invention is based on a negative replica method. More in detail, the present method comprises the steps of:
1) preparing an aqueous slurry of a ceramic material;
2) mixing the slurry with a liquid, viscous organic phase to obtain a dough, wherein the organic phase is substantially insoluble in water, and is thermally decomposable into gaseous residues;
3) drying the dough; and
4) removing the organic phase by thermal decomposition.

As has been mentioned, the present method has the advantage that a ceramic body is obtained which has a porous structure consisting of interconnected pores. Moreover, a particularly high porosity can be achieved while maintaining superior mechanical properties.

In addition, it has been found that a ceramic body obtainable by the present method possesses a specific microporous surface inside the macropores. In other words, the surface of the ceramic body, including the surface within the pores, has a certain advantageous rugosity. By virtue of this feature, a good attachment of cells is obtained when cells are seeded onto the body, e.g. in tissue engineering applications. Also, by virtue of this feature the ceramic body may give rise to osteoinduction. The above described ceramic materials prepared by a slip-casting method have been found not to have this feature.

The ceramic material of which the slurry is prepared can in principle be any material of which it is desired to prepare a porous body of. In other words, the choice for a particular ceramic material will depend on the objective application of the final product. In view of the envisaged application of the porous body as a scaffold in tissue engineering, in accordance with the invention it is preferred that the ceramic material is a calcium phosphate. Highly preferred calcium phosphates may be chosen from the group of octacalcium phosphate, apatites, such as hydroxyapatite and carbonate apatite, whitlockites, α-tricalcium phosphate, β-tricalcium phosphate, sodium calcium phosphate, and combinations thereof.

Under certain circumstances it may be desired to incorporate an additive in the aqueous slurry. Examples of such additives are binders, surfactants, pH controlling agents, deflocculants, and the like. As binder, a water soluble polymer may be used, such as a cellulose derivative (e.g. carboxymethylcellulose) may be used, preferably in an amount of between 0.05 and 0.5 wt.%, based on the weight of the slurry. A pH controlling agent may suitably be employed to control the solubility of the ceramic material. It will be clear that it is to be avoided that (a significant amount of) the ceramic material dissolves in the aqueous phase. The skilled person will be able to determine whether there is a need for the use of a certain additive, based on his general knowledge.

The concentration of the ceramic material in the slurry will depend on the solubility of the chosen ceramic material in water. Generally, said concentration will be chosen between 50 and 80 wt.%, preferably between 55 and 75 wt.%, based on the weight of the slurry. The slurry may be prepared by admixing water and the ceramic material under stirring until a homogeneous slurry is obtained.

As has been mentioned above, the slurry is mixed with an organic phase, which does not dissolve in the slurry (i.e. is substantially insoluble in water) and does not react chemically with the ceramic material in the slurry.

In order to enable a homogeneous distribution of the organic phase throughout the slurry, the organic phase needs to be in a liquid form at the time of mixing. Further, in order to ensure that the resultant mixture has the form of a moldable dough, allowing shaping of the resulting ceramic body, the organic phase should be viscous. It is also possible that the viscosity of the organic phase is relatively low at the time of mixing with the slurry, but increases upon drying.

Another requirement that has to be met by the organic phase is that it should be removable from the dough by thermal decomposition. It is preferred that the organic phase decomposes into volatile and/or gaseous residues upon exposure to temperatures above 200°C or 400°C. Depending on the envisaged application of the ultimate ceramic body, it is desirable that substantially no charred or tar-like residues are formed upon thermal decomposition, respectively remain within the porous structure of the ceramic body after sintering.

Suitable examples of materials that can be used to form the organic phase will readily be identified by the skilled person, based on the considerations presented above. Depending on the material chosen, an organic solvent may or may not be present. It will be clear that, if an organic solvent is to be used, it should be chosen such that it does not substantially interfere with the characteristics of the organic phase recited above.

Without wishing to be exhaustive, the following suitable materials for use in the organic phase can be mentioned: waxes, shellac, fatty acids, fats, epoxy resins, polyurethane resins, polyester resins, poly(meth)acrylate resins and combinations thereof. Particular good results have been obtained using a poly(meth)acrylate resin. Such a resin can be a homo- or a copolymer of various acrylate and/or methacrylate monomers. Preferably, the resin is polymethylmethacrylate.

When a synthetic polymer is used in the organic phase, it has been found advantageous to incorporate a small amount of monomeric material. This monomeric material may polymerize *in situ* in the dough formed by mixing the organic phase with the aqueous slurry of the ceramic material, thereby beneficially affecting the formation of interconnected pores in the final ceramic body. The monomeric material may be included in a ratio of up to 3:1, with respect to the weight of the polymer. Preferred ratios range from 1:1 to 1:2.5 with respect to the weight of the polymer. If necessary, a small amount of a suitable catalyst or initiator enabling the polymerization may be present.

In case it is desired to prepare a ceramic body having a particularly high porosity, a foaming agent may conveniently be included in the organic phase. The foaming agent may be present in the organic phase in amounts of up to 10 wt.%. By use of a foaming agent, it has been found possible to achieve a porosity of about 60%. A preferred example of a foaming agent is a combination of sodium bicarbonate and citric acid, which agents may suitably be employed in a weight ratio of between 1:2 and 1:5. Advantageously, this specific foaming agent substantially only acts when brought into contact with water (i.e. with the aqueous slurry of ceramic material). This has the effect that the organic phase will swell to a certain extent, while said phase will maintain its continuity, so that the mechanical properties of the ceramic body that is being prepared are substantially not adversely affected as a result of the use of a foaming agent.

In another embodiment, combustible particulate matter, such as pine tree branches or rigid polymeric fibers, are incorporated in the organic phase, in the slurry of ceramic material or in the mixture of the organic phase and said slurry. This matter is meant to decompose when the organic phase is removed by thermal decomposition. As a result, a ceramic body is obtained which has discrete cavities in its structure which have the shape and size of the particulate matter that has been removed. Such cavities may advantageously have the shape of tunnels, of channels, which facilitates the flow of culture medium through the ceramic body when it is used as a scaffold in tissue engineering and cells seeded onto it are being cultured.

The aqueous slurry of ceramic material and the organic phase are preferably mixed in a volume ratio of between 1:2 and 3:1, more preferably between 1:1 and 2:1. The dough will preferably comprise at least 35 vol.% of the organic phase, remainder preferably being the aqueous slurry. Both components of the dough may be mixed in any manner, taking care to obtain a homogeneous mixture.

The dough is subsequently dried. It is at this stage that a polymerization reaction may take place in the organic phase, as such is aimed at. Preferably the drying is carried out for a duration of at least 5 hours at atmospheric conditions. If a more thorough drying is desired, a microwave can be used for this purpose. It is further preferred that the dough is molded into a desired shape prior to drying. To this end, the dough may be brought into a mold, which can for instance be a polypropylene or polyethylene mold, or any other material that can be removed by burning substantially without release of toxic gases.

The dried dough is subsequently placed in a furnace in order to thermally decompose, and remove, the organic phase. Suitable conditions will depend on the nature of the organic phase. Typically, thermal decomposition will be achieved at a temperature between 200 and 800°C. In order to ensure that the organic phase substantially completely disappears, the heating may be prolonged for a duration of up to 24 or even 36 hours.

After the thermal decomposition step, a porous ceramic body is obtained which may find application in itself. For many applications, however, it is preferred that the ceramic body is sintered. Sintering may be performed at a temperature between 800 and 1400°C, preferably between 1000 and 1300°C.

The thus obtained ceramic body has superior mechanical properties. In particular, it has a very high strength. The compressive strength will preferably be at least 10 MPa. Furthermore, the ceramic body has a porosity of a very advantageous configuration. The pores are interconnected and preferably have a mean diameter ranging from 400 to 4000 µm. The lower limit of the porosity of the ceramic body may be 40%, or even as high as 50% or 70%.

The above properties make the ceramic body highly suitable for use as a scaffold in tissue engineering. In this regard, the term tissue engineering is intended to refer to any process wherein cells are seeded onto the scaffold material and cultured there, either *in vitro* or *in vivo,* to form tissue of a desired type. For the formation of tissue, cells of various types may be used ranging from stem cells to all sorts of differentiated cells. Due to its mechanical properties, the present porous ceramic body is particularly useful for tissue engineering bone tissue or for repair of defects at non-load bearing sites, but also at load bearing sites.

It has furthermore been found that the present ceramic body may be crushed to form a granulate of a desired porous structure. The granulate so obtained may find application in for example oral surgery and plastic surgery of the face, as well as in spine surgery and orthopedics. Preferably, the mean diameter of the particles of the granulate are between 2 and 3.5 mm.

The invention will now be elucidated by the following, non-restrictive example.

### EXAMPLE

### Chemicals used:

Hydroxylapatite powder (HA), Merck
Polymethmethacrylate (PMMA)
Carboxy methylcellulose (CMC)
Dolapix from Zschimmer & Schwarz Gmbh
Ammonia solution (25%) Merck
Polymethylmethacrylate (PMMA) powder (Dental Biolux International
Methylmethacrylate (MMA) monomer
Dibenzoylperoxide (DBPO, usually of 75 % purity)
N,N dimethyl - p - toluidine (DMPT).

A slurry was prepared by admixing the ingredients listed in table 1 in the specified amounts. Stirring was continued until a homogeneous slurry was obtained.

**Table 1 Composition of HA slurry**

| Ingredient | Quantity (g) | Weight (%) |
|---|---|---|
| Demi-H₂O | 49 | 28.6 |
| NH₄OH (25%) | 4.5 | 2.6 |
| Dolapix CE 64 | 2.5 | 1.5 |
| HA calcined | 115 | 67.1 |
| CMC | 0.26 | 0.15 |
| Total | 171.2 | |

An organic phase was prepared by mixing a powder and a liquid component in respective amounts of 9.3 grams and 3.7 grams. The components were added to each other in a Teflon beaker and stirred until a homogeneous mixture was obtained. The nature of both components is specified in table 2.

**Table 2 Composition of PMMA**

| Component | |
|---|---|
| POWDER component | PMMA Powder + 1% Dibenzoylperoxide (DBPO, usually of 75 % purity) |
| LIQUID component | Methylmethacrylate (monomer) + 2% N,N dimethyl - p - toluidine (DMPT). |

Next, 35.0 grams of the HA slurry and the organic phase were mixed under moderate stirring. The volume ratio of HA slurry to organic phase was 6:4. The organic phase was allowed to set at room temperature (RT). The resulting composite green body was dried in air for 6 hours.

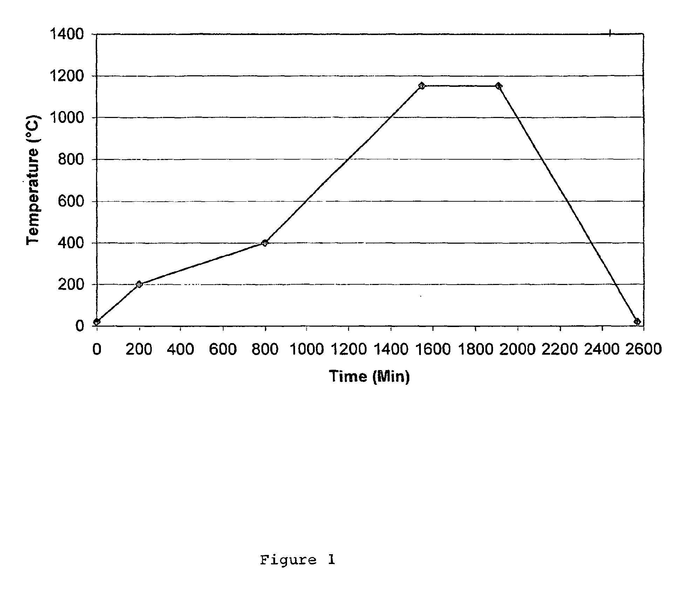

Finally, the dried composite green body was placed in a furnace and heated and sintered according to the heating profile shown in figure 1.

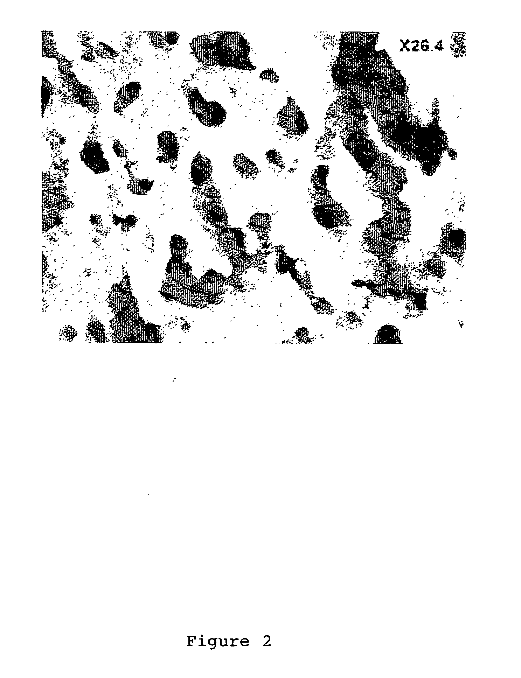

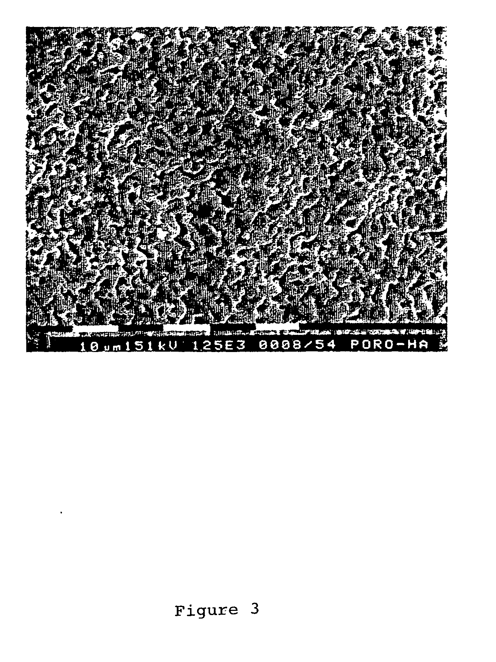

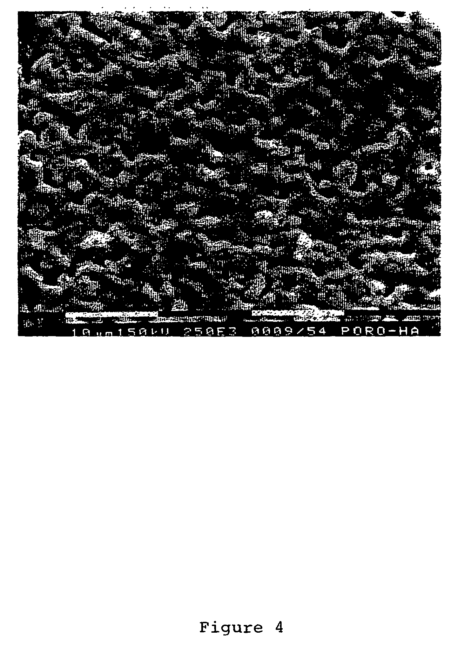

Of the obtained ceramic body, microscopic photographs were taken which are shown in figures 2, 3 and 4. Figure 2 shows the structure under an optical microscope with a magnification of 26.4. Figure 3 shows the microstructure of the ceramic body within the pores at a magnification of 1250, and figure 4 shows the same microstructure at a higher magnification (2500).

## Claims

1. Process for preparing a porous ceramic body comprising the steps of:
1) preparing an aqueous slurry of a ceramic material;
2) mixing the slurry with a liquid, viscous organic phase to obtain a dough, wherein the organic phase is substantially insoluble in water, and is thermally decomposable into gaseous residues;
3) drying the dough; and
4) removing the organic phase by thermal decomposition.

2. Process according to claim 1, wherein the ceramic material is a calcium phosphate.

3. Process according to claim 2, wherein the calcium phosphate is chosen from the group of octacalcium phosphate, apatites, such as hydroxyapatite and carbonate apatite, whitlockites, α-tricalcium phosphate, β-tricalcium phosphate, sodium calcium phosphate, and combinations thereof.

4. Process according to any of the preceding claims, wherein the slurry comprises between 50 and 80 wt.% of ceramic material, based on the weight of the slurry.

5. Process according to any of the preceding claims, wherein the organic phase comprises a material chosen from the group of waxes, shellac, fatty acids, fats, epoxy resins, polyurethane resins, polyester resins, poly(meth)acrylate resins, and combinations thereof.

6. Process according to claim 5, wherein the organic phase comprises a poly(meth)acrylate resin.

7. Process according to any of the preceding claims, wherein the organic phase solidifies upon drying.

8. Process according to claims 6-7, wherein the organic phase comprises polymethylmathacrylate, methylmethacrylate and a polymerizing initiator.

9. Process according to any of the preceding claims, wherein the dough is dried for at least 5 hours under atmospheric conditions.

10. Process according to any of the preceding claims, wherein the organic phase is removed at a temperature of between 200 and 800°C.

11. Porous ceramic body obtainable by a process according to any of the preceding claims, having interconnected pores of a mean size between 400 and 4000 µm, a porosity of at least 40%, and a compressive strength of at least 10 MPa.

12. Use of a porous ceramic body according to claim 11 as a scaffold in tissue engineering.

13. Process according to any of the preceding claims, wherein the porous ceramic body is sintered at a temperature of between 800 and 1400°C.

14. Porous ceramic body obtainable by a process according to claim 13, having interconnected pores of a mean size between 400 and 4000 µm, a porosity of at least 40%, and a compressive strength of at least 10 MPa.

15. Use of a porous ceramic body according to claim 14 as a scaffold in tissue engineering.

16. Process for preparing a porous ceramic granulate comprising crushing a porous ceramic body according to claims 11 or 14.

17. Porous ceramic granulate obtainable by a process according to claim 16 having a mean particle diameter of between 2 and 3.5 mm and interconnected pores of a mean size between 400 and 4000 µm, a porosity of at least 40%, and a compressive strength of at least 10 MPa.

## Patentansprüche

1. Verfahren zur Herstellung eines porösen Keramikkörpers, umfassend die Schritte:
1) Herstellung einer wässrigen Aufschlämmung aus einem Keramikmaterial;
2) Mischen der Aufschlämmung mit einer flüssigen viskosen organischen Phase, um einen Teig zu erhalten, wobei die organische Phase im Wesentlichen in Wasser unlöslich ist und thermisch zu gasförmigen Rückständen zersetzt werden kann;
3) Trocknen des Teiges und
4) Entfernen der organischen Phase durch thermische Zersetzung.

2. Verfahren nach Anspruch 1, bei dem das Keramikmaterial ein Calciumphosphat ist.

3. Verfahren nach Anspruch 2, bei dem das Calciumphosphat ausgewählt wird aus der Gruppe Octacalciumphosphat, Apatite wie Hydroxyapatit und Carbonatapatit, Whitlockite, α-Tricalciumphosphat, β-Tricalciumphosphat, Natriumcalciumphosphat und deren Kombinationen.

4. Verfahren nach einem der vorstehenden Ansprüche, bei dem die Aufschlämmung zwischen 50 und 80 Gew.-% Keramikmaterial, bezogen auf das Gewicht der Aufschlämmung, ausmacht.

5. Verfahren nach einem der vorstehenden Ansprüche, bei dem die organische Phase ein Material umfasst, ausgewählt aus der Gruppe Wachse, Schellack, Fettsäuren, Fette, Epoxidharze, Polyurethanharze, Polyesterharze, Poly(meth)acrylatharze und deren Kombinationen.

6. Verfahren nach Anspruch 5, bei dem die organische Phase ein Poly(meth)acrylatharz umfasst.

7. Verfahren nach einem der vorstehenden Ansprüche, bei dem die organische Phase beim Trocknen fest wird.

8. Verfahren nach Anspruch 6 bis 7, bei dem die organische Phase Polymethylmethacrylat, Methylmethacrylat und einen Polymerisationsinitiator umfasst.

9. Verfahren nach einem der vorstehenden Ansprüche, bei dem der Teig unter atmosphärischen Bedingungen mindestens 5 Stunden-getrocknet wird.

10. Verfahren nach einem der vorstehenden Ansprüche, bei dem die organische Phase bei einer Temperatur zwischen 200 und 800°C entfernt wird.

11. Poröser Keramikkörper, der gemäß einem der vorstehenden Ansprüche erhältlich ist und miteinander verbundene Poren mit einer durchschnittlichen Größe zwischen 400 und 4000 µm, eine Porosität von mindestens 40 % und eine Druckfestigkeit von mindestens 10 MPa aufweist.

12. Verwendung eines porösen Keramikkörpers nach Anspruch 11 als Gerüst beim künstlichen Aufbau von Gewebe.

13. Verfahren nach einem der vorstehenden Ansprüche, bei dem der poröse Keramikkörper bei einer Temperatur zwischen 800 und 1400°C gesintert wird.

14. Poröser Keramikkörper, der durch ein Verfahren gemäß Anspruch 13 erhältlich ist und miteinander verbundene Poren mit einer durchschnittlichen Größe zwischen 400 und 4000 µm, eine Porosität von mindestens 40 % und eine Druckfestigkeit von mindestens 10 MPa aufweist.

15. Verwendung eines porösen Keramikkörpers nach Anspruch 14 als Gerüst für den künstlichen Aufbau von Gewebe.

16. Verfahren zur Herstellung eines porösen Keramikgranulats, umfassend das Zerstoßen eines porösen Keramikkörpers gemäß Anspruch 11 oder 14.

17. Poröses Keramikgranulat, das nach einem Verfahren gemäß Anspruch 16 erhältlich ist und einen mittleren Teilchendurchmesser zwischen 2 und 3,5 mm und miteinander verbundene Poren mit einer durchschnittlichen Größe zwischen 400 und 4000 µm, eine Porosität von mindestens 40 % und eine Druckfestigkeit von mindestens 10 MPa aufweist.

## Revendications

1. Procédé de préparation d'un corps céramique poreux comprenant les étapes de:
1) préparation d'une suspension aqueuse d'un matériau céramique ;
2) mélange de la suspension avec une phase organique visqueuse liquide afin d'obtenir une pâte dans laquelle la phase est sensiblement insoluble dans l'eau, et est thermiquement décomposable en résidus gazeux ;
3) séchage de la pâte ; et
4) élimination de la phase organique par décomposition thermique.

2. Procédé selon la revendication 1, dans lequel le matériau céramique est un phosphate de calcium.

3. Procédé selon la revendication 2, dans lequel le phosphate de calcium est choisi dans le groupe du phosphate d'octacalcium, des apatites, tels qu'un hydroxyapatite et un carbonate-apatite, des whitlockites, du phosphate d'α-tri-calcium, du phosphate de β-tricalcium, du phosphate de sodium calcium et de leurs combinaisons.

4. Procédé selon l'une quelconque des revendications précédentes, dans lequel la suspension comprend entre 50 et 80 % en poids de matériau céramique, basé sur le poids de la suspension.

5. Procédé selon l'une quelconque des revendications précédentes, dans lequel la phase organique comprend un matériau choisi dans le groupe des cires, des gommes laques, des acides gras, des graisses, des résines époxy, des résines polyuréthanes, des résines polyesters, des résines poly(méth)acrylates, et des combinaisons de ceux-ci.

6. Procédé selon la revendication 5, dans lequel la phase organique comprend une résine poly(méth)acrylate.

7. Procédé selon l'une quelconque des revendications précédentes, dans lequel la phase organique se solidifie lors du séchage.

8. Procédé selon les revendications 6 et 7, dans lequel la phase organique comprend un polyméthylméthacrylate, un méthylméthacrylate et un initiateur de polymérisation.

9. Procédé selon l'une quelconque des revendications précédentes, dans lequel la pâte est séchée pendant au moins 5 heures dans les conditions atmosphériques.

10. Procédé selon l'une quelconque des revendications précédentes, dans lequel la phase organique est enlevée à une température située entre 200 et 800 °C.

11. Corps céramique poreux pouvant être obtenu par un procédé selon l'une quelconque des revendications précédentes, ayant des pores interconnectés d'une taille moyenne allant de 400 à 4000 µm, une porosité d'au moins 40 % et une résistance à l'écrasement d'au moins 10 MPa.

12. Utilisation d'un corps céramique poreux selon la revendication 11, comme matrice en ingénierie tissulaire.

13. Procédé selon l'une quelconque des revendications précédentes, dans lequel le corps céramique poreux est fritté à une température située entre 800 et 1400 °C.

14. Corps céramique poreux pouvant être obtenu par un procédé selon la revendication 13, ayant des pores interconnectés d'une taille moyenne allant de 400 à 4000 µm, une porosité d'au moins 40 % et une résistance à l'écrasement d'au moins 10 MPa.

15. Utilisation d'un corps céramique poreux selon la revendication 14, comme matrice en ingénierie tissulaire.

16. Procédé de préparation d'un granulé céramique poreux comprenant l'écrasement d'un corps céramique poreux selon les revendications 11 à 14.

17. Granulé céramique poreux pouvant être obtenu par un procédé selon la revendication 16, ayant un diamètre moyen de particules allant de 2 à 3,5 mm et des pores interconnectés d'une taille moyenne allant de 400 à 4000 µm, une porosité d'au moins 40 % et une résistance à l'écrasement d'au moins 10 MPa.
